# EUROPEAN PATENT APPLICATION

(11) **EP 1 275 962 A1**
(43) Date of publication of application: **15.01.2003**
(21) Application number: 01830469.1
(22) Date of filing: 13.07.2001
(51) Int. Cl.: G01N 31/22, G01N 33/84, G01N 31/00, G01N 33/52

(54) **Method for detecting oxidizing substances or free radicals in saliva and corresponding kit**

(71) Applicant: Technopharma SA, 6900 Lugano (CH)
(72) Inventor: Epifani, Vita, 6900 Lugano (CH)
(74) Representative: Banchetti, Marina

(57) **Abstract**

Method for the detection of oxidative substances or free radicals in saliva, that allows identifying the presence of oxidative species and/or free radicals in the organism via a simple test based on a colorimetric method, carried out on a saliva sample. The invention also includes a specially designed testing kit for implementing said method, and may be applied for self-diagnosing oxidative stress states.

## Description

The present invention concerns a test for detecting oxidative substances or free radicals in saliva. More particularly, this invention relates to a diagnostic method that allows detecting the presence of oxidative species and/or free radicals in the organism by a simple test carried out on a sample of saliva, and that can thus be applied for the self-testing of oxidative stress states. The invention also includes the testing kit specially designed for use with this method.

In aerobe organisms, the oxygen that is absolutely essential for their survival is also directly or indirectly responsible for oxidative damage caused by its reactive metabolites. As is known, the term "reactive oxygen species", or ROS (or also "reactive oxygen metabolites", or ROM), is currently used to refer to all free radicals and non-radical chemical species that currently take part in oxidative biological processes and whose excess with respect to normal equilibrium conditions is considered to be the basis for an ever-increasing number of degenerative processes and pathologies. Specifically, the term ROS includes the superoxide anionic radical, O₂⁻•, the hydroxy radical, OH•, the singlet oxygen, ¹O₂, and hydrogen peroxide, H₂O₂, as well as the alkoxy radicals, RO•, and peroxy radicals, ROO•, that are formed from organic molecules in oxidative processes. The production of these metabolites mainly depends on physical events, such as UV radiation, or chemical events, such as xenobiotic agents.

Irrespective of their formation process, these metabolites are harmful to the organism for their high reactivity, and their action affects various cell components, among which a considerable number of structural proteins, enzymes and amino acids, DNA and RNA, carbohydrates as well as lipids and phospholipids - both membrane and lipoprotein ones. In particular, this oxidative damage reduces the anti-oxidative capacity of human seric albumin and, above all, damages the lipids of the cytoplasmic membrane (lipid peroxidation). Moreover, owing to the reactivity of these chemical species, the base metabolic processes are also affected.

In particular, the lipid peroxidation of cell membranes is closely correlated to cell aging (Sato, T. et al., *Gerontology,* 38, 50, **1992**; Miyazawa, T. et al., *Lipids*, 28, 789, **1993**; Suzuki, T. et al., *Lipids,* 28, 775, **1993**) and to the development of various pathologies (Pryor, W.A., *Photochem. Photobiol.* 28, 787, **1978**; Oarada, M. et al., *Biochem. Biophys. Acta,* 960, 229, **1988**; Oarada, **M**. et al., *Biochem. Biophys. Acta,* 1012, 156, **1989**). Furthermore, studies conducted to find any correlation between the quantity of oxidative substances and/or free radicals present in an organism and the onset of illnesses have shown that several pathologies, such as atherosclerosis (Ester-bauer, H. et al., *J. Lipid Res*., 28, 495, **1987**; Miyazawa, T., *Free Radical Biol. Med.,* 7, 209, **1989**; Miyazawa, T. et al., *Biomed. Chromatogr.,* 4, 131, **1990**), various neoplastic diseases, above all affecting the epithelia (sarcomas) (Yoshida, L.S. et al., *Lipids,* 25, 565, **1990**; Sato, T. et al., *Mutat. Res.,* 251, 91, **1991**; Ozturk, H.S., et al., *Cancer Biochem. Biophys.,* 16 (1-2), 157, **1998**, Goldstein, B.D., Witz, G., *Free Radical Res. Commun.,* 11, 3, **1990**), certain infections (Kondo, Y. et al., *Biochem. Biophys. Acta,* 1127, 227, **1992**), Alzheimer's disease (Jeandel, C. et al., *Gerontology,* 35 (5-6), 275, **1989**), and the increased predisposition to bone fractures in smokers (Meihus, H. et al., *J. Bone Miner Res.,* 14 (1), 129, **1999**), are all strictly correlated to the production of these reactive metabolites and to the inability on the part of the organism's natural defences (or those acquired through the diet) to extinguish them.

In a situation of this type, i.e. the excessive production of reactive oxygen metabolites, the organism faces a process known as "oxidative stress".

In order to defend itself from the damage caused by the said metabolites, the organism has developed anti-oxidative defence systems to extinguish the reactivity of these substances. These systems may be of a proteic nature, such as seric albumin (HSA), superoxide dismutase (SOD), catalase, lactoperoxidase or small peptides, or of a non-proteic nature, such as ubiquinolols, anthocyanins, flavonoids, liposoluble vitamins (vitamins A and E) or hydrosoluble vitamins (vitamin C) - vitamin intake normally occurring through food. In particular, a diet that is rich in natural anti-oxidative substances helps the body to neutralise the excess oxidative substances or free radicals, thus preventing the development of the aforesaid pathologies. On the other hand, a diet that is poor in substances that can extinguish the reactive oxygen metabolites, or smoking or alcohol abuse, not only increase the production of these oxidative substances, but also diminish the capacity of the aforesaid endogenous substances to extinguish the metabolites.

The known remedies against oxidative stress that are useful in the prevention of all the pathologies connected thereto may be largely classified in the following two groups:
- the intake of natural anti-oxidative substances through a fruit and fibre rich diet.
- the use of pharmaceutical preparations containing doses corresponding to the body's daily requirements of liposoluble and hydrosoluble vitamins and of metals that can extinguish oxidative substances and/or free radicals.

Unfortunately, the uncontrolled intake of certain substances has some drawbacks. For example, epidemiological studies show that an excessive intake of vitamin A and the like causes neurotoxicity (Snodgrass, S.R., *Mol. Neurobiol*., 6 (1), 41, **1992**). Another example is to be found in the uncontrolled intake of selenium, high doses of which increase the incidence of tumours of the liver, pancreas and skin (Birt, D.F., *Proc. Soc. Exp. Biol. Med.,* 183 (3), 311, **1986**). These components are normally found in different amounts according to the type of formulation, in commercially available preparations in the therapeutic category of "multivitamin complexes".

In view of these drawbacks the ideal solution would therefore be to monitor the actual anti-oxidative or radical extinguishing capacity of endogenous systems. In the event of a deficit due to altered metabolic factors that may be correlated to oxidative stress in general, to age, situations of phlogosis in general or to certain pathologies, it should be possible to adequately support the body's anti-oxidative capacity by rebalancing the "stock" necessary for extinguishing the excessive production of oxidative substances.

The first technique designed and used for detecting peroxides in the organism was the colorimetric technique with thiobarbituric acid (TBA test; Kohn, H.I. and Liversedge, *M*., *J. Pharmacol. Exp. Ther*., 83, 292, **1944**). Over the years, the refinement of this technique, the industrial development of more accurate analytical detection instruments and the use of this technique in conjunction with HPLC techniques has allowed developing increasingly more effective systems, at a qualitative and quantitative level, for the separation and identification of oxidative substances and/or free radicals (Yamamoto, Y. et al., *Anal. Biochem*., 160, 7, **1987**; Yamamoto, Y. and Ames, B.N., *Free Radical Biol. Med.,* 3, 359, **1987**; Frei, B. et al., *Anal. Biochem.,* 175, 120, **1988**; Yamamoto, Y. and Niki, E., *Biochem. Biophys. Res. Commun.,* 165, 988, **1989**; Halliwell, B. and Gutteridge, J.M.C., "Free Radicals in Biology and Medicine", Oxford University Press (Clarendon), Oxford, **1989**).

Although being very accurate, the aforesaid peroxide detection methods are not without drawbacks that discourage their current use by unskilled personnel and prevent their use by patients themselves. In particular, such drawbacks are linked to the following aspects:
- these methods are applied on biological samples that are difficult to obtain, such as blood, serum or cell extracts;
- they require specialised personnel, laboratories and instrumentation.

The availability of an efficient and simple diagnostic method which can be used even by non-specialist persons and which can detect the presence or any excess of oxidative substances, by analysing an easily available body fluid, can allow detailed studies to be made on the aforesaid equilibrium, assessing how it may change in relation to normal physiological conditions or in the presence of pathologies. It can also allow regulating the intake - if and when necessary - of anti-oxidative substances in order to make good any deficits in the endogenous defence mechanisms.

There are essentially three body fluids that can be used in this kind of test: saliva, sweat and urine. The latter, as is known, is the excretion of the body's metabolic waste and, as such, may not be useful for the purpose. An analysis carried out on body sweat may be invalidated by the kind of atmosphere present when conducting the test. Saliva, on the other hand, due to its biochemical composition and to the fact that it represents the first area of prevention against the intake and production of oxidative substances when eating, and the place where these substances are extinguished, plays a crucial role in maintaining normal overall physiological conditions and particularly of the digestive system, as well as of the upper airways.

As is known, saliva is a biological fluid that not only contains typically anti-oxidative substances such as ascorbic acid, vitamin E and uric acid (Frei, B. et al., *Proc. Natl. Acad. Sci. U.S.A.,* 85, 9748, **1988**; Frei, B. et al., *Proc. Natl. Acad. Sci. U.S.A.,* 86, 6377, **1989**; Niki, E et al., *J. Nutr. Sci. Vitaminol.,* 34, 507, **1988**; Ames, B.N. et al., *Proc. Natl. Acad. Sci. U.S.A.,* 78, 6858, **1981**), but also enzymes, such as lactoperoxidase, that can convert hydroperoxide compounds into the corresponding stable hydroxyl derivatives.

Therefore, in view of the aforesaid considerations and due to its characteristics and easier testing with respect to other biological fluids, saliva lends itself well to a screening of the balance between anti-oxidative substances and oxidative substances that may be present in it because they are produced in the same organism or taken in through food. Thus, determining the presence of oxidative substances may advantageously provide a valid diagnostic aid in order to detect any metabolic disorders and to monitor the therapeutic efficacy of any anti-oxidative pharmacological action taken.

Thus, within the frame of the present invention a method has been devised for directly detecting oxidative substances present in saliva. Since the method does not require blood samples or any sophisticated analytical techniques, it can be carried out independently by anybody and thus actually allows self-testing. The method is based on a reactive scheme which is in general lines similar to the peroxide detection methods already described in the literature - i.e. on the oxidation of the iodide ion to molecular iodine by the oxidative substances present in saliva, and on the visualisation of the resulting iodine via the formation of a coloured complex with starch-water.

A testing kit has also been devised for applying the above method, containing all the elements for carrying out the analysis. Such kit allows considerable ease of use and makes testing and diagnosis formulation accessible to anyone.

Thus, the present invention specifically provides a method for the detection of oxidative substances in saliva, comprising the following operations:
- incubating, in an acidic environment, a saliva sample together with an amount of a iodide of an alkali metal or alkaline-earth metal sufficient to react with any oxidative substances present in said saliva sample;
- adding an amount of starch-water sufficient to form a coloured complex with any molecular iodine that developed from the previous operation;
- detecting the presence, if any, of a more or less bright blue colouring in the solution resulting from the previous operation.

The alkali metal iodide - or alkaline-earth metal iodide - to be preferred for carrying out these operations is potassium iodide.

Preferably, the method should use about 0.5 ml of saliva, about 0.01 mmoles of potassium iodide and 1 mg of starch-water; or corresponding multiples of the above quantities. The acidic environment is preferably created by adding a weak acid and this can be provided in the aforesaid preferred formulation by adding about 40 mg of citric acid or a multiple of said quantity corresponding to the quantities of said potassium iodide, saliva and starch-water.

Further, the present invention specifically provides a testing kit for the detection of oxidative substances in saliva, including the following items:
- a first container suitable to house all the reagents and containing an amount of iodide of an alkali metal or alkaline-earth metal sufficient to react with any oxidative substances present in a sample of saliva of fixed volume to be submitted to analysis;
- a second container with a weak acid, and
- a third container with a sufficient amount of starch-water to form a coloured complex with any molecular iodine that has formed during the test.

Preferably, as noted, said iodide of an alkali metal or alkaline-earth metal should be potassium iodide. In a preferred embodiment of the invention, the first container is a test-tube containing about 0.01 mmoles of potassium iodide; the second container is the stopper of the test-tube itself, in the form of a reservoir-stopper equipped with perforations allowing the weak acid to flow out into the test-tube, and containing about 40 mg of citric acid; the third container, which is preferably a separate disposable container, will contain about 100 µl of a 1 % aqueous solution of starch-water.

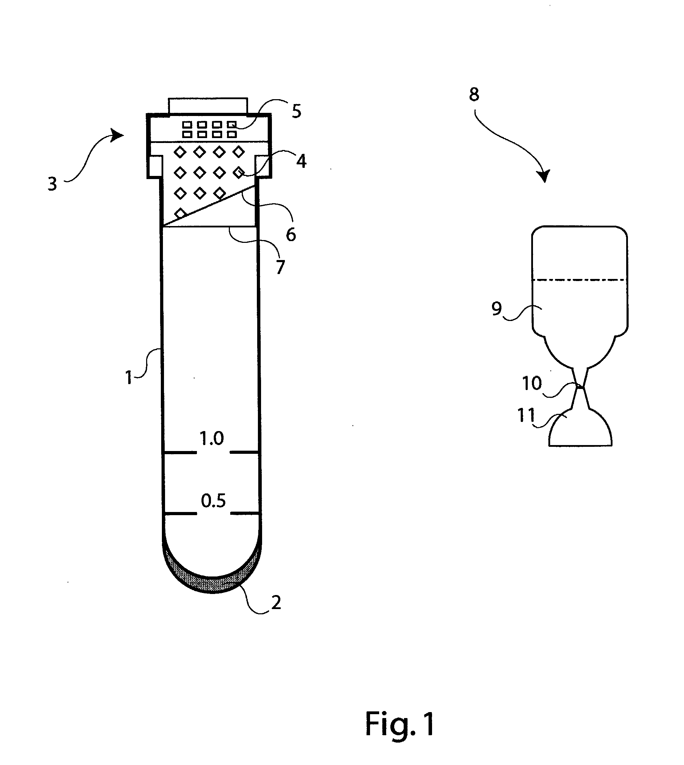

A preferred embodiment of the present invention will now be described in the following examples as mere illustrations, with reference to the attached Figure 1, which shows a testing kit for carrying out the method of the invention.

### EXAMPLE 1

### Kit structure and composition

A testing kit for carrying out the test proposed according to the present invention, illustrated in Figure 1, is typically composed of the following items.
1. A test-tube (1) containing the lyophilized powder (2) of 100 µl of an aqueous solution 0.1 M of potassium iodide; the stopper (3) of the test-tube (1) is designed in the form of a reservoir-stopper and contains 40 mg of citric acid in the form of crystalline powder (4) hermetically sealed from the external environment. A compartment of the reservoir-stopper - located above the one containing the citric acid powder (4) - contains silica powder (5) whose function is to maintain the citric acid powder (4) in anhydrous conditions. The reservoir-stopper has a sharp slanting edge (6) that on use will cut the lamina (7) of the stopper, so that the citric acid powder (4) can drop into the test-tube (1).
2. A disposable container (8) containing 100 µl of a 1% aqueous solution (9) of starch-water. The example container (8), made of plastic material, can be opened by cutting along the line (10) of least resistance and removing the closing element (11).

The kit must be kept in a dry place away from light sources until it is used.

### EXAMPLE 2

### Method for the detection of oxidative substances

The test is carried out in the following way:
- a saliva sample, with no prior treatment, is introduced in the test-tube (1) described in Example 1, up to the reference mark corresponding to a volume of 0.5 ml;
- after closing the test-tube (1), the upper section of the reservoir-stopper is pressed in order to release the citric acid powder (4) into the test-tube (1);
- after shaking for one minute, the 100 µl of 1% aqueous solution (9) of starch-water, contained in the disposable container (8), is poured into the test-tube (1) and the resulting suspension is manually shaken for a few seconds.

In these conditions, any presence of free radicals or oxidative substances in saliva will turn the iodide into iodine which, in the presence of starch-water, will form a bright blue coloured complex. The test outcome is considered positive when the suspension takes on a more or less bright blue colour, which signals the presence of free radicals or oxidative substances; on the other hand, if the suspension remains colourless, the result is considered negative: i.e. the analysed saliva does not contain oxidative substances.

More specifically, the colouring obtained is given a value of +, ++ or +++ depending on whether it is faint, distinct or very bright.

It must be stressed that the test should not be carried out if there are any lesions in the upper section of the oropharyngeal apparatus or when suffering from influenza because the presence of blood or inflammation can lead to false positive results.

### EXAMPLE 3

### Experimental results of the test

The test contained in the present invention, described in the two aforesaid examples, has been carried out on a total of 100 individuals controlled for a period ranging from a minimum of 30 days to a maximum of 150 days. The individuals were 48 females and 52 males, aged 22-35 years.

In view of the fact that oxidative stress varies considerably between individuals, and even within the same individual, owing to the onset of physio-pathological events of even short duration, each individual was monitored in the phase leading up to the administration of the synthetic anti-oxidative substances. This monitoring was sufficiently long (about two weeks) to allow to determine a statistically significant reference value as the "average value of the test".

In almost all the cases examined it was possible to correlate the anomalous peak values obtained in the test with concomitant objectively detectable causes. There does not seem to be any significant difference in response to the test between individuals of different genders, except as regards the physiological aspect of the menstrual cycle, which involves an increase in oxidative stress.

Among the physio-pathological events that appear to correlate with high test values there are: a) influenza state; b) the active phase of a Herpes s. infection; c) the menstrual cycle; d) reduced sleep; e) neuro-psychic stress.

When the examined individuals were given a diet supplemented with vitamin C, 1 g per day for 1-2 weeks, most of them (92%) then had significantly lower values (≤ 40%) than the average value they themselves had reported during the monitoring phase without taking the synthetic anti-oxidative substances. In only a few cases (8%) the intake of vitamin C did not produce any variation with respect to the average test value.

It is interesting to note that, in cases where there was a clear response to the vitamin C treatment, the new reduced oxidative stress conditions were found for a period ranging from 5 to 15 days after the treatment period itself.

The present invention has been disclosed with particular reference to some specific embodiments thereof, but it should be understood that modifications and changes may be made by the persons skilled in the art without departing from the scope of the invention as defined in the appended claims.

## Claims

1. A method for the detection of oxidative substances in saliva, comprising the following operations:
• incubating, in an acidic environment, a saliva sample together with an amount of a iodide of an alkali metal or alkaline-earth metal sufficient to react with any oxidative substances present in said saliva sample;
• adding an amount of starch-water sufficient to form a coloured complex with any molecular iodine that developed from the previous operation;
• detecting the presence, if any, of a more or less bright blue colouring in the solution resulting from the previous operation.

2. A method according to claim 1, wherein said iodide of an alkali metal or alkaline-earth metal is potassium iodide.

3. A method according to claim 2, wherein said amount of potassium iodide is about 0.01 mmoles, the amount of the saliva sample is about 0.5 ml and said amount of starch-water is about 1 mg, or said amounts are corresponding multiples of the indicated amounts.

4. A method according to any one of claims 1-3, wherein said acidic environment is provided by the addition of a weak acid.

5. A method according to claim 4, wherein said weak acid is citric acid.

6. A method according to claim 3, wherein said acidic environment is provided by the addition of about 40 mg of citric acid, or of a multiple of said amount corresponding to said amounts of potassium iodide, saliva and starch-water.

7. A testing kit for the detection of oxidative substances in saliva, including the following items:
• a first container (1) suitable to house all the reagents and containing an amount of iodide (2) of an alkali metal or alkaline-earth metal sufficient to react with any oxidative substances present in a sample of saliva of fixed volume to be submitted to analysis;
• a second container (3) with a weak acid (4); and
• a third container (8) with a sufficient amount of starch-water (9) to form a coloured complex with any molecular iodine that has formed during the test.

8. The testing kit according to claim 7, wherein said iodide (2) of an alkali metal or alkaline-earth metal is potassium iodide.

9. A testing kit according to claims 7 or 8, wherein said first container (1) is a test-tube and said second container (3) is the stopper of the test-tube itself, in the form of a reservoir-stopper equipped with perforation means (6) allowing the weak acid (4) to flow out into the test-tube (1).

10. A testing kit according to any one of claims 7-9, wherein said first container (1) contains about 0.01 mmoles of potassium iodide, said second container (3) contains about 40 mg of citric acid and said third container (8) contains about 100 µl of a 1 % aqueous solution of starch-water.
